(19) **Europäisches Patentamt / European Patent Office / Office européen des brevets**

(11) **EP 4 156 200 A1**

(12) **EUROPEAN PATENT APPLICATION**

(43) Date of publication:
**29.03.2023 Bulletin 2023/13**

(21) Application number: **21199049.4**

(22) Date of filing: **27.09.2021**

(51) International Patent Classification (IPC):
**G16H 30/40** (2018.01)  **G16H 50/20** (2018.01)
**A61B 5/055** (2006.01)

(52) Cooperative Patent Classification (CPC):
**G16H 50/20; A61B 5/055; G16H 30/40**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universitätsmedizin der Johannes Gutenberg-Universität Mainz 55131 Mainz (DE)**

(72) Inventors:
• **Gonzalez-Escamilla, Gabriel 64521 Groß-Gerau (DE)**
• **Groppa, Sergiu 55127 Essenheim (DE)**

(74) Representative: **Lucke, Andreas Boehmert & Boehmert Anwaltspartnerschaft mbB Pettenkoferstrasse 22 80336 München (DE)**

(54) **METHOD FOR RECONSTRUCTING A BRAIN NETWORK**

(57) According to the disclosure, a method for reconstructing a brain network (205), comprises providing, in a memory of a computing device, an image of at least a section of a subject's brain (201); determining, in a processing unit of the computing device, a three-dimensional volume model (202) of the section of the subject's brain (201); segmenting, by the processing unit, the volume model (202) into a plurality of sub-volumes, each sub-volume corresponding to an anatomical subfield of the subject's brain (201) and having a volume value; for each of the sub-volumes, determining, by the processing unit, a modified volume for the sub-volume, wherein determining said modified volume comprises calculating said modified volume based on the volume value of the sub-volume and variables multiplied with constants, wherein the constants are constants determined for a population, comprising a plurality of individuals, to which the subject belongs; for each combination of two sub-volumes, determining, by the processing unit, a similarity between the sub-volumes, based on a comparison of the respective modified volume for each of the sub-volumes; and determining, by the processing unit, a brain network (205) for the section of the subject's brain (201). The brain network (205) comprises nodes (206), each node (206) representing one of the sub-volumes, and edges (207), each edge (207) connecting two nodes (206) and being indicative of the determined similarity between the sub-volumes. At least one of the steps from the group of storing the brain network (205) in the memory of the computing device, displaying the brain network (205) on a display unit of the computing device, and sending the brain network (205) to a remote computing device via a transmission unit of the computing device is performed.

Further, a method for monitoring a brain network, a computing device, a computer program product and a computer-readable storage medium are provided.

Fig. 2

**Description**

FIELD OF THE INVENTION

The present invention is in the field of biomedical research and clinical diagnostics. In particular, the invention relates to a method for reconstructing a brain network, in particular based on clinically available anatomical MRI sequences, as well as to a corresponding method for monitoring a brain network, a computing device, computer program product and computer-readable storage medium.

BACKGROUND

The study of the brain's intrinsic connectivity patterns, in particular the interactive assessment of the topological architectural composition between different anatomical regions of the brain, has become increasingly important in recent years. The network topology of the brain is fundamental to the study of motor, sensory, and cognitive processes, as well as the mechanisms underlying various clinical pathologies, including the most common forms of neurodegenerative diseases (such as Alzheimer's Disease, Parkinson's disease, and frontotemporal dementia, among others) and other neurological entities (for example multiple sclerosis, epilepsy, stroke, etc.).

Since its introduction, graph theory has become a potent research instrument that allows modeling brain networks as well as evaluating how different brain regions orchestrate different functions. Such networks have different structural and functional components, and damage to key components can cause an increase in the whole network's vulnerability to lesions or neuropathology, including neurodegeneration.

Evidence that the modelled brain network has a good correspondence to neural correlates comes mainly from studies with simultaneous derivation of functional imaging (fMRI and EEG) and significant overlap with structural connectivity patterns (i.e., white matter connections) derived from diffusion imaging data (cf. Greicius et al.: Resting-state functional connectivity reflects structural connectivity in the default model network, Creb. Cortex, 19: 72-78, 2009). However, the understanding on how interactions between functional and structural connectivity patters sustain the system or augment its vulnerability to disease remains to be unveiled. This can be attributed to the fact that both functional and diffusion imaging data have relatively poor spatial resolution in comparison to anatomical MRI data, such as Ti-weighted imaging.

Currently, brain networks at the individual level can be derived from diffusion imaging, depicting white matter fiber tracts that structurally connect regions, or from functional imaging, depicting covarying patterns of neural activity. Both of these modalities allow the reconstruction of brain networks given their multi-volume nature. In this case, several image acquisitions are performed in serial during a single session. However, neither of these imaging modalities can capture brain atrophy nor neurodegenerative processes in full. Therefore, the search of algorithms for network reconstructions at the individual level from Ti-weighted imaging has gained attention (see for example Batalle et al.: Normalization of similarity-based individual brain networks from gray matter MRI and its association with neurodevelopment in infants with intrauterine growth restriction, Neuroimage, 83: 901-911, 2013; Kong et al.: Mapping individual brain networks using statistical similarity in regional morphology from MRI, PLoS ONE, 10: e0141840, 2015; Li et al.: Construction of individual morphological brain networks with multiple morphometric features, Front. Neuroanat., 11: 34, 2017). In this sense, networks at the individual level derived from Ti-weighted images, so-called morphometric networks, remain as the intermediate between structural connectivity and functional connectivity by depicting the amount of anatomical (or morphometric) similarity between brain regions.

Despite recent advances in the development of algorithms to reconstruct individual level networks from Ti-weighted images, currently available methods are based on the covariance across several morphometric measures derived from the Ti-weighted images. However, the combination of morphometric measures ignores their intrinsic relationships. For example, with advancing age, the cortical thickness decreases and sulci become deeper in many brain regions, which results in an increase in the surface area of the cortex. In this sense, the interpretability of the reconstructed networks is not straightforward. A further challenge with regard to the interpretation arises because some methods go beyond T1-weighted data and reconstruct the networks by adding a mixture between morphometric measures and diffusion imaging derivatives (cf. Seidlitz et al.: Morphometric Similarity Networks Detect Microscale Cortical Organization and Predict Inter-Individual Cognitive Variation, Neuron, 97(1), 231-247.e7, 2018). This, in turn increases the complexity of the network reconstruction and hampers the interpretability of the outcome connectivity.

A method that uses a single morphometric measure derived from T1-weighted data, is based on the generation of three-dimensional cubes of a predefined size along the brain. The cubes are then rotated depending on their location in order to account for the shape of the brain, i.e. folding and curvature. Following, the volume of the voxels contained inside each cube, in its final position, is used to compute the covariance between all pairs of cubes (Tijms et al.: Similarity-based extraction of individual networks from gray matter MRI scans, Cereb. Cortex, 22: 1530-1541, 2012). This method is parcellation-free, which is an advantage for whole brain connectivity analyses. However, a disadvantage of such approach using cubes is that it is very unlikely that simple rotations would make the cubes fit with the complex folding of the brain, leaving some regions uncovered and other regions possibly covered by more than one cube. This problem of fitting cubes escalates when evaluating subcortical structures, since their shape is very different from that of the cortex and some structures may be smaller than the proposed cube size of 5 mm$^3$ to 6 mm$^3$.

Overall, despite recent advances, no straightforward way to derive network measures at the single subject level from Ti-weighted images currently exist.

## SUMMARY OF THE INVENTION

It is thus an object of the invention to provide improved technologies for reconstructing a brain network, in particular a method to reconstruct individual networks from Ti-weighted data based on single morphometric measures.

This object is met by a method for reconstructing a brain network according to claim 1, a method for monitoring a brain network according to claim 11, a computing device according to claim 12, a computer program product according to claim 13 and a computer-readable storage medium according to claim 14. Embodiments of the present invention are detailed in the dependent claims.

According to an aspect, a method for reconstructing a brain network is provided. The method comprises providing, in a memory of a computing device, an image of at least a section of a subject's brain. In a processing unit of the computing device, a three-dimensional volume model of the section of the subject's brain is determined. The volume model is segmented, by the processing unit, into a plurality of sub-volumes, each sub-volume having a volume value and corresponding to an anatomical subfield of the subject's brain. For each of the sub-volumes, a modified volume for the sub-volume is determined by the processing unit. Determining said modified volume comprises calculating said modified volume based on the volume value of the sub-volume and variables multiplied with constants, wherein the constants are constants determined for a population, comprising a plurality of individuals, to which the subject belongs. For each combination of two sub-volumes, a similarity between the sub-volumes is determined by the processing unit, based on a comparison of the respective modified volume for each of the sub-volumes. By the processing unit, a brain network for the section of the subject's brain is determined, the brain network comprising nodes, each node representing one of the sub-volumes, and edges, each edge connecting two nodes and being indicative of the determined similarity between the sub-volumes. At least one of the following steps is performed: storing the brain network in the memory of the computing device, displaying the brain network on a display unit of the computing device, and sending the brain network to a remote computing device via a transmission unit of the computing device. The brain network reconstructed using the method may be considered an individualized brain network for the subject at least a section of whose brain the method is performed for. Consequently, when considering the possibility to apply the method to brains of different subjects, the method may be considered a method for reconstructing individualized brain networks.

According to another aspect, a method for monitoring a brain network is provided. The method for monitoring a brain network comprises performing the method for reconstructing a brain network at a first point in time, thereby reconstructing a first brain network, performing the method for reconstructing a brain network at a second point in time, the second point in time being later than the first point in time, thereby reconstructing a second brain network, and comparing the first brain network to the second brain network. Herein, the first brain network and the second brain network are reconstructed for the same section of the same subject's brain.

According to an additional aspect, a computing device is provided, the computing device comprising a processing unit and a memory, and preferably further comprising a display unit and/or a transmission unit. The computing device is configured to perform the method for reconstructing a brain network.

According to a further aspect, a computer program product is provided comprising instructions which, when the program is executed by a computing device, cause the computing device to carry out the method for reconstructing a brain network.

A computer-readable storage medium according to yet another aspect comprises instructions which, when executed by a computing device, cause the computing device to carry out the method.

With respect to the brain network, the edges may also be considered vertices in the sense known in connection with graph theory. The term edge is used herein to differentiate the edges or vertices of the brain network from vertices as commonly used in connection with MRI, in particular with regard to computing MRI-derived metrics from vertices.

The section of the subject's brain may be any section of interest, in particular for research or diagnosis purposes, with regard to functional connectivity of anatomical subfields of such brain section. Anatomical subfields of the brain or a section thereof are in particular defined in the relevant art of medical sciences based on their functionality within the brain.

The computing device may be any device suitable for executing the method. For example, the computing device may be a personal computer, tablet computer, PDA, smartphone or any other suitable device. The processing unit may be any processing unit capable of performing said steps of the method. The processing unit may be a single processing unit or, alternatively, may be a distributed processing unit. For example, the processing unit may be formed with a central processing unit (CPU) and a graphical processing unit (GPU) of the computing device and different steps of the method may be performed by different sub-units of the processing unit, i.e. the CPU and GPU in the case of the above example. In particular, method steps for which a GPU is better suited because of its particular architecture may performed by the GPU and other method steps may be performed by the CPU.

In the context of the present disclosure, a three-dimensional volume model of a brain or a section of a brain is a volumetric model, in particular a model made up of voxels, that represents the brain or section thereof at least with regard to its volume and shape in three-dimensional space. The three-dimensional model may be an approximation model, i.e. the volume and shape of the brain or brain section need not be represented by the three-dimensional model exactly but within error boundaries within which the three-dimensional model is still useful for the purpose at hand as defined herein.

The three-dimensional volume model is determined by the processing unit from the image of at least a section of the subject's brain. The three-dimensional volume model may in particular be determined by deducing volumetric properties of the brain or brain section from the image of the at least section of the subject's brain. For example, the image maybe an image acquired by biomedical imaging techniques known as such. The three-dimensional volume model may be determined based on a plurality of images of the at least section of the subject's brain. In particular, three-dimensional volumetric information for the three-dimensional volume model may be determined from a plurality of images containing two-dimensional information. For example, images of different planes of the subject's brain or a section thereof may be used for reconstructing a three-dimensional volume model of the brain or brain section. In a preferred embodiment, the three-dimensional volume model is determined from a plurality of images which do not all show the same target (e.g. plane of volumetric section of the brain) captured at different times. In particular, the three-dimensional volume model may be determined without use of images acquired by way of functional imaging (such as fMRI). For determining the three-dimensional volume model, reconstruction techniques which are per se known in the art maybe employed.

Correspondingly, the sub-volumes are segments of the three-dimensional volume model and are as such defined by their shape, arrangement within the three-dimensional volume model and a volume value, i.e. an amount of three-dimensional space occupied by the sub-volume. Accordingly, the sum of the volume values of the sub-volumes equals the total volume of the three-dimensional volume model, and therefore the total volume of the at least a section of the subject's brain, within given error margins due to errors in imaging and subsequent processing steps.

The modified volume for each of the sub-volumes is determined based on the respective volume value, i.e. a metric of an actual amount of physical space occupied by the respective sub-volume. Due to the modifications caused by calculating the modified volume, the modified volume may no longer represent an actual physical volume, i.e. amount of occupied space. In particular, the modified volume may be a property of the sub-volume's connectivity to other sub-volumes such that a high similarity of two values for the modified volume is indicative of a high connectivity of sub-volumes exhibiting such two values of their modified volumes. At the same time, the modified volume may still have the mathematical properties of a volume.

By determining a modified volume for each of the sub-volumes, each sub-volume may be adjusted for factors indicated by the variables such that the sub-volumes are rendered independent from individual effects reflected by such factors, such as age heterogeneity, scanner type or head size.

The modified volume may be determined, for each of the sub-volumes, according to the following equation:

$$\mathrm{vol}_{\mathrm{mod}} = \beta_0 + \beta_1 \cdot x_1 + \ldots + \beta_N \cdot x_N + \epsilon$$

Herein, $\mathrm{vol}_{\mathrm{mod}}$ may be the modified or estimated volume, $\beta_0$ may be a model section or offset, $x_1 \ldots x_N$ may be the variables, $\beta_1 \ldots \beta_N$ may be coefficients determined by multiplying the volume value of the sub-volume with the respective constants determined for a population for each of the variables, and $\epsilon$ may be a remaining error. Any or all of the variables $x_i$ (i.e. $x_1 \ldots x_N$) may be variables on a continuous spectrum, such as a volume value, or a discontinuous spectrum, such as an age in years. Any or all of the variables $x_i$ may be discrete or categorical variables, for example a value chosen from a list associated with a property not associated with a spectrum, such as a denomination of an anatomical subfield.

In preferable embodiments, some or all of the variables $x_i$ (i.e. $x_1 \ldots x_N$) may be chosen from the group consisting of the age of the subject, the total volume of the section of the subject's brain, a variable indicative of the sub-volume for which the modified volume is determined, and the gender of the subject. In particular, the variables $x_i$ may be chosen such that they represent factors the variation of which between individuals was determined to have a significant impact on connectivity of a given sub-volume in an individual. The variables $x_i$ may be chosen based on their significance such that variables with high significance are included to assure adequate accuracy for reconstructing the brain network while variables with lower significance (but still having an impact on connectivity) may be excluded to assure adequate performance when the method is executed using a given computing device and/or to assure adequate performance in determining the constants (in particular before the method for reconstructing a brain network is performed). Thereby, a balance between accuracy and efficiency in view of a particular computing device or group (or class) of computing devices may be achieved.

In an exemplary embodiment, for each of the sub-volumes, the modified volume may be determined according to the following formula:

$$vol_{mod} = \beta_0 + \beta_1 \cdot x_1 + \beta_2 \cdot x_2 + \beta_3 \cdot x_3 + \beta_4 \cdot x_4 + \epsilon$$

wherein $vol_{mod}$ is the modified volume, $\beta_0$ is a model section or offset, $x_1$ is the age of the subject, $x_2$ is the total volume of the section of the subject's brain, $x_3$ is a variable indicative of the sub-volume for which the modified volume is determined, $x_4$ is a variable indicative of the gender of the subject, $\beta_1$, $\beta_2$, $\beta_3$, $\beta_4$ are the respective coefficients relating to the volume value and the respective constant determined for a population for each of the variables, and $\epsilon$ is a remaining error.

The constants used in determining the modified volume may be obtainable in a statistical analysis and/or using a training algorithm, preferably using linear regression.

In embodiments relating to coefficients $\beta_1 ... \beta_N$, the constants to which the coefficients $\beta_1 ... \beta_N$ relate may be obtainable using linear regression, preferably according to a general linear model. In particular, the constants may be obtainable by fitting a general linear regression model or general linear model, known in the art as such, with the variables and extracting model residuals as modified volume values.

In this case, residuals may be defined by the deviance of the general linear regression model, wherein the deviance indicates the extent to which the likelihood of the saturated model exceeds the likelihood of the proposed model, the saturated model being a model that perfectly fits the data, in the sense that the fitted responses ($\hat{Y}_i$ - $vol_{mod}$ below) is equal to the observed responses ($Y_i$ - sub-volume). If the proposed model has a good fit, the deviance will be small, but if the proposed model has a bad fit, the deviance will be high. Thus, the deviance may be a measure of the model error ($\epsilon$) and the deviance residuals may represent the contributions of individual samples to the total model deviance.

Herein, the response variable ($vol_{mod}$) may be modelled in function (or as a combination) of the explanatory variables or predictors ($x_i$) and an intercept ($\beta_0$) plus the error term (as outlined with regard to deviance above). The intercept may be a unit term (i.e. a vector of 1's of the length $vol_{mod(n)}$, where n is the number of observations), and may represent a baseline level used as reference to allow other predictors to model estimations relative to it. Since $\beta_0$ has a constant value (i.e., multiplication with 1 does not alter a value), the constants may be obtainable by fitting the general linear regression model (or solving the model formula) by finding a link function (g) between the distribution mean parameter ($\mu$) of the response variable and a linear function of each explanatory variable x ($\eta$) for each observation, such as:

$$\eta_n = g(\mu_n)$$

Thereby, the coefficients $\beta_1 ... \beta_N$, i.e. the constants to which said coefficients relate, may be obtainable for a given population. In this case, the final $vol_{mod}$ is finally computed as a weighted sum of all link functions (or reference functions), quantifying its potential contribution in explaining the observed responses. Ultimately, $vol_{mod}$ may be seen as the projection of the observed values to the estimated values, also known as predicted values. In other words, at some time previous to performing the method, fitting or training may be performed using linear regression to determine $\beta_0 ... \beta_N$ and $\epsilon$ such that they may be applied in the formula as outlined above to determine the respective modified volume when performing the method.

In alternative embodiments, in addition or as an alternative, the constants may be obtainable using other statistical and/or training methods, for example using a generalized linear model and/or an artificial neural network.

In this context, a method for determining constants for use in the method for reconstructing a brain network is provided. The method comprises applying a statistical method and/or a training (or fitting) algorithm, preferably a general linear model, to a formula for calculating the modified volume in the method for reconstructing a brain network, thereby determining the constants. With regard to the method for determining constants, in particular for applying a general linear model, the details outlined above in connection with constants obtainable in a statistical method and/or using a training algorithm, preferably a general linear model, may apply accordingly.

For each combination of two sub-volumes, the similarity between the sub-volumes may be determined according to the formula

$$C_{m(i,j)} = 1 / (1 + (vol_{mod-i} - vol_{mod-j}))$$

Herein, $C_{m(i,j)}$ may be the similarity between a sub-volume i and a sub-volume j, $vol_{mod-i}$ may be the modified volume of the sub-volume i, and $vol_{mod-j}$ may be the modified volume of the sub-volume j. Thereby, a morphometric difference

$$d_{(i,j)} = vol_{mod-i} - vol_{mod-j}$$

between the sub-volumes i and j may be determined and the similarity may be determined based on the morphometric difference according to the formula

$$C_{m(i,j)} = 1 / (1 + d_{(i,j)}).$$

The section of the subject's brain may be the Hippocampus. In this case, the sub-volumes are sections of the hippocampus. In particular embodiments, each hemisphere of the Hippocampus may be segmented into 12 sections such that the volume model is segmented into 24 sub-volumes. In an exemplary embodiment, the 12 sections for each hemisphere of the hippocampus may respectively correspond to the anatomical subfields of the hippocampal tail, the cornu ammonis 1 (CA1), the cornu ammonis 2/3 (CA2/3), the cornu ammonis 4 (CA4), the subiculum, the presubiculum, the granule cell layer of the dentate gyrus (DG), the hippocampus-amygdala transition area (HATA), the parasubiculum, the molecular layer of the hippocampus, the fimbria and the hippocampal fissure.

In embodiments, other sections of the subject's brain (including superstructures or substructures of the hippocampus) may be used in the method for reconstruction of a brain network in addition or as an alternative to the hippocampus. For example, the volume model may be determined for the thalamus, the epithalamus, the pineal gland, the hypothalamus, the pituitary gland, the subthalamus, the amygdala, further limbic structures and/or the entire brain. In embodiments in which the volume model is determined for a section of the subject's brain including the hippocampus, one of the sub-volumes into which the volume model is segmented may correspond to the hippocampus.

In general, selection of a brain section may depend on the MRI quality and the parcellation algorithms used. For example, MRI acquisitions at 7T or 3T with small voxel sizes (such as sizes smaller than 1 mm and high SNR) may be particularly suitable for assessing smaller and deeper structures. Clinical scans (such as 3T with voxel sizes greater or equal to 1 mm and middle to low SNR) may be particularly suitable for studying small deep brain gray matter structures, such as the basal forebrain or the nucleus subthalamicus.

The image of the section of the subject's brain may be an image obtained by MRI (magnetic resonance imaging) of the subject's brain. In a preferred embodiment, the image of the section of the subject's brain is an image referring to anatomical MRI data, for example obtained using Ti-weighted (Ti-w) imaging, i.e. imaging based on spin-lattice relaxation. Alternatively or additionally, the image may be obtained by computer tomography (CT), ultrasound (US) scanning, molecular imaging, such as positron emission tomography (PET), and/or neurotransmitter imaging, such as magnetic resonance spectroscopy (MRS or NMR). In particular embodiments, the three-dimensional volume model may be determined based on a plurality of images obtained using different imaging modalities.

The image provided in the memory of a computing device may be an image in MRI space or, alternatively, may be an image that has been transformed into standard space. Herein, the terms MRI space and standard space refer to the coordinate system of the images, in particular with regard to image size, orientation, coordinate origin, and resolution. MRI space is intrinsic to the acquired images, with image properties depending on acquisition parameters and may differ from subject to subject, for example according to head size and position inside the scanner magnet. The term standard space implies a transformation (or deformation) to match a particular orientation, origin and size that is to be (almost) identical in all subjects. Standard space may in particular be used for performing voxel-wise statistics, in which assessing the same brain location across subjects while accounting for head size is necessary. The variables and constants may depend on the image space. For example, an adjustment based on variables referring to head size may be used in the method if an image in MRI space is used but may be unnecessary and therefore omitted if an image transformed into standard space is used.

Said segmenting may be performed using a probabilistic algorithm. In embodiments, the image may be parcellated using a probabilistic atlas. Such probabilistic atlas may be (previously) built by manual labelling on a training dataset resulting in a point-to-point correspondence between all training subjects. Such atlas may provide the probability of each brain region to belong to a given voxel, the probability of each brain region given the classification of neighboring voxels (neighborhood function), and the probability distribution function of voxel intensities, modelled as a normal distribution, for each brain region at each voxel. The image of at least a section of the subject's brain may then be parcellated by normalizing such image to the common space and incorporating the subject-specific voxel intensities to find the optimal parcellation that maximizes the probability of observing the input data. The segmenting the volume model into a plurality of sub-volumes may comprise applying a Bayesian inference approach and a probabilistic high-resolution ex-vivo atlas based on previously scanned subjects and in vivo MRI data. In an exemplary embodiment, the volume model is segmented by applying a Bayesian inference approach and a probabilistic high-resolution (~0.1 mm isotropic) ex-vivo atlas based on 15 subjects scanned at 7T (manual delineation of the hippocampal substructures) and in vivo MRI data (manual annotation of the adjacent extra-hippocampal structures), wherein the probabilistic atlas has an isotropic resolution of 0.13 mm. Thereby, delineating the sub-volumes to a high degree of accuracy with regard to the anatomical subfields may be enabled.

Storing the brain network may comprise compiling the determined similarities between the sub-volumes into a squared adjacency matrix. Such squared adjacency matrix, which may also be called connectivity matrix, may represent the connectivity strength across network regions. The squared adjacency matrix may be useful for evaluating network topology and/or determining regions with connectivity abnormalities related to disease conditions. Generally, storing the brain network may comprise converting information of the nodes and edges in a non-graphical representation, such as a squared adjacency matrix, that allows reconstructing a graphical representation of the nodes and edges of the brain network for displaying the brain network on a display at a later point in time.

Displaying the brain network may comprise displaying the edges as connecting lines between the nodes, wherein a graphical property of the edges is indicative of the determined similarity between the sub-volumes, the graphical property being selected from an edge thickness, an edge color, an edge length, an edge brightness, and an edge structure. The determined similarity between two given sub-volumes may be indicated by more than one graphical property of the corresponding edge. For example, each edge may be displayed with a thickness and color each corresponding to the determined similarity of the nodes connected by the edge. An edge structure may, for example, be a line structure, i.e. the edge is displayed as a line with a certain structure, such as a solid line, a dotted line, a dashed line, a dot-and-dash line, a zigzag line, a double line, etc.

In addition to a graphical property of an edge being indicative of the determined similarity between connected sub-volumes, a further graphical property of such edge may be indicative of another factor of interest, for example a physical proximity of anatomical subfields represented by the nodes connected by the edge.

Similarly, a graphical property (e.g. node size, node color, node brightness, node structure, such as hollow dot, filled dot, double dot, etc.) of each node may be indicative of a factor of interest of the given node. For example, a color of a given node may indicate the anatomical subfield represented by the node while the nodes indicative of the same anatomical subfield in different brain hemispheres or lobes may be distinguished by a node structure.

For each of the sub-volumes, by the processing unit, a connectivity measure indicative of the connectivity of the sub-volume to the other sub-volumes may be determined, the connectivity measure may be compared to a representative connectivity measure of the population to which the subject belongs, and a significance value for the sub-volume may be determined, wherein the significance value is indicative of a deviation of the connectivity measure for the sub-volume from the representative connectivity measure. In such embodiments, said displaying the brain network may comprise displaying a three-dimensional representation of the section of the subject's brain, wherein each sub-volume is displayed and a graphical property of each sub-volume is indicative of the determined significance value for the sub-volumes, the graphical property being selected from a sub-volume color, a sub-volume brightness, a sub-volume opacity, and a sub-volume structure. A sub-volume structure may, for example, be a structure of a surface of the sub-volume, such as a rough, knobby or otherwise structured surface, or a (graphical) segmentation of the sub-volume, such as a representation as an arrangement of spheres, cubes, polyhedra or the like. Each of the sub-volumes may be displayed as a three-dimensional representation of the anatomical subfield to which a given sub-volume corresponds.

In general, the similarity between sub-volumes may be indicative of a connectivity between the sub-volumes and the brain network may be representative of the connectivities between the different sub-volumes of the section of the subject's brain. Therefore, the brain network may be useful in assessing connectivities between anatomical subfields of the subject's brain and may be used in clinical research and/or diagnosis. For example, methods of graph theory may be applied to the brain network to evaluate how the brain restructures in the context of a certain disease or in response to a specific type of therapy, including invasive and non-invasive stimulation.

The embodiments described above with regard to the method for reconstructing a brain network may apply to the method for monitoring a brain network, the computing device, the computer program product and/or the computer-readable storage medium, accordingly.

The method for monitoring a brain network may comprise accordingly performing the method for reconstructing a brain network at one or more additional points in time, each of such additional points in time being later than the previous points in time, reconstructing additional brain networks. Some or all of the resulting brain networks, including the first brain network, the second brain network and the additional brain network or networks, may then be compared to each other. With regard to the method for monitoring a brain network, comparing the resulting networks (i.e. the second brain network and any additional brain networks) with the first one (and with one another), may deliver the possibility to evaluate brain reorganization mechanisms in connection with disease progression, as well as help in assessing the response to therapeutic interventions (invasive and non-invasive). A difference in time between points in time at which the method for reconstructing a brain network is performed in the method for monitoring a brain network may be, for example, several days or in the scope of one or more months or years, depending on the expected speed of change in the brain network.

A system for reconstructing a brain network may be provided, the system comprising the computing device according to the disclosure, an imaging device configured to acquire the image of at least a section of a subject's brain and optionally a remote computing device, separate from the computing device and configured to receive the brain network sent from the computing device via a receiving unit of the remote computing device. Preferably, the imaging device is an MRI device. Alternatively, the imaging device may be a CT machine, ultrasound machine, PET machine, NMR machine, or other suitable imaging device. The system may comprise a plurality of imaging devices of different types for acquiring a plurality of images of at least a section of a subject's brain.

LIST OF FIGURES

In the following, a detailed description of the invention and exemplary embodiments thereof is given with reference to the figures. The figures show schematic illustrations of

Fig. 1: a flow chart of a method for reconstructing a brain network;

Fig. 2: output generated by a method for reconstructing a brain network;

Fig. 3: a group comparison of network metrics obtained from individual morphometric networks; and

Fig. 4: associations between a clustering coefficient and cognitive performance of a subject.

DESCRIPTION OF THE PREFERRED EMBODIMENTS

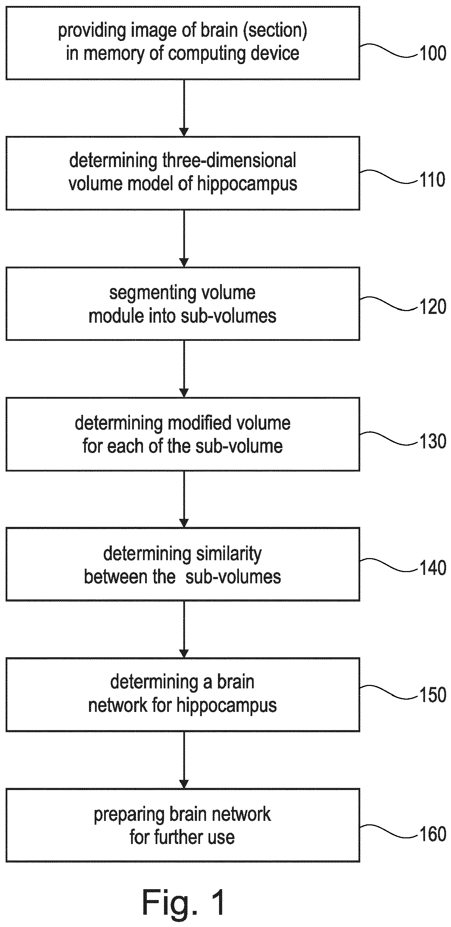

Fig. 1 shows a flow chart of a method for reconstructing a brain network. In step 100, an image of at least a section of a subject's brain is provided in a memory of a computing device. In the exemplary embodiment of Fig. 1, the image is in fact a plurality of Ti-weighted MRI images of different planes of the subject's hippocampus. The MRI images have been previously acquired using imaging techniques known as such and were previously stored in the form of image data in the hard disk as part of a memory of the computing device which, in the example, is a personal computer (PC). The image data is then transferred to a RAM of the PC, forming a further part of the memory of the computing device, whereby the image is provided in the memory of the computing device for the performance of the following steps.

Following, in step 110, a three-dimensional volume model of the hippocampus of the subject's brain is determined in a processing unit of the computing device. In the exemplary embodiment, the processing unit is a distributed processing unit formed by all processing units provided in the PC, which thereby form processing subunits. In particular, such processing sub-units include a CPU of the PC and a GPU of a graphics card of the PC. In an example, the main computing load of the determining of the volume model is taken by the GPU, which is efficient for graphical calculations, with additional computing performed by the CPU. In determining the three-dimensional volume model, the MRI images are analyzed and three-dimensional data for the hippocampus is calculated from the image data. A volume model in line with the three-dimensional data is then built, the three-dimensional volume model consisting of voxels arranged to represent the shape (outer delineation, folding, curvature, etc.) and physical volume of the brain. Thereby, the three-dimensional volume model is a three-dimensional representation of the subject's hippocampus, including the shape and volumetric content of the hippocampus.

Next, the three-dimensional volume model is segmented into sub-volumes in step 120. In particular, the volume model of the hippocampus is segmented into 24 sub-volumes, namely 12 sub-volumes per hemisphere, each of the 12 sub-volumes per hemisphere corresponding to one of the following anatomical subfields of the hippocampus: hippocampal tail, cornu ammonis 1 (CA1), cornu ammonis 2/3 (CA2/3), cornu ammonis 4 (CA4), subiculum, presubiculum, granule cell layer of the dentate gyrus (DG), hippocampus-amygdala transition area (HATA), parasubiculum, molecular layer of the hippocampus, the fimbria and the hippocampal fissure. In the particular example, the segmenting is performed using the software Freesurfer 6.0 running on the computing device (explained in more detail below), but any suitable algorithmic method, in particular probability-based methods, may be used for the segmenting step 120. The segmenting step 120 results in 24 sub-volumes, each of which has a volume value, i.e. a numerical value that indicates the volumetric content of the sub-volume, for example in units of mm$^3$.

In step 130, a modified volume is determined for each of the sub-volumes. This is achieved by calculating the modified volume based on the volume value of the respective sub-volume and on variables multiplied with constants. Herein, the constants have been previously determined for a population to which the subject for whom the brain network is reconstructed belongs. Thereby, the constants are applicable to the subject based on the population to which the subject belongs and in contrast to other populations to which the subject does not belong.

The modified volume is still in the mathematical dimension of a volume but does no longer represent an actual volume value of the given sub-volume. Rather, the modified volume is a metric of the connectivity in terms of brain function of the sub-volume with other sub-volumes. In this context, the modified volume is similar for sub-volumes that exhibit a high degree of interconnectivity among each other and dissimilar for sub-volumes with a low degree of interconnectivity.

In a specific example, the modified volume may be determined according to the formula

$$\mathrm{vol_{mod}} = \beta_0 + \beta_1 \cdot x_1 + \beta_2 \cdot x_2 + \beta_3 \cdot x_3 + \beta_4 \cdot x_4 + \epsilon$$

In this example, $\mathrm{vol_{mod}}$ is the modified volume, $\beta_0$ is a model section or offset and $\epsilon$ is a remaining error. $x_1$ is the age of the subject, $x_2$ is the total volume of the section of the subject's brain, $x_3$ is a variable indicative of the sub-volume for which the modified volume is determined, and $x_4$ is a variable indicative of the gender of the subject. $\beta_1$, $\beta_2$, $\beta_3$ and $\beta_4$ are coefficients each resulting from multiplying the volume value of the respective sub-volume with the respective constants ($k_1$, $k_2$, $k_3$, $k_4$) determined for a population for each of the variables.

It is noted that, for the different variables, the relevant population to which the subject belongs is different. While, regarding the age of the subject, the relevant population is the population of the same age as the subject (or alternatively the population belonging to the same of a predetermined number of age ranges as the subject), the relevant population with regard to the section of the subject's brain is the overall population, as all of the population has such brain section. The variables have been selected so as to provide a relevant value for the modified volume with regard to connectivity of the sub-volumes while ensuring adequate performance (i.e. limiting necessary computing power) when the method is performed on a given computing device and/or in determining the constants. It follows, that the variables selected and constants determined may vary depending on specific metrics or details of interest, on the computing device or devices used, as well as on the outcome of alternative or future research on which the selection and determination maybe based.

In an exemplary embodiment, the coefficients $\beta_1$, $\beta_2$, $\beta_3$ and $\beta_4$ - in particular the constants $k_1$, $k_2$, $k_3$, $k_4$ — have been determined beforehand by performing regression according to a general linear model to the formula, based on data pertaining to the respective populations. The model is thereby fitted, the constants $k_1$, $k_2$, $k_3$, $k_4$ being determined as a scalar value, such that at the time of performing the method for reconstructing a brain network, the modified volume is calculated by inserting the volume value, the values of the variables $x_1$, $x_2$, $x_3$, $x_4$ and the values of the constants $k_1$, $k_2$, $k_3$, $k_4$ (wherein $\beta_1$ = volume value · $k_1$; $\beta_2$ = volume value · $k2$; $\beta_3$ = volume value · $k_3$; $\beta_4$ = volume value · $k_4$) into the formula and solving for $vol_{mod}$ according to the formula.

The modified volumes of each of the sub-volumes are then used in step 140 in determining a similarity between the sub-volumes. This is achieved by comparing the modified volume of each combination of two sub-volumes. In the specific example, the similarity is determined using the formula

$$Cm_{(i,j)} = 1 / (1 + d_{(i,j)}).$$

Herein, $d_{(i,j)}$ is the morphometric difference between regions i and j and is calculated according to the formula

$$d_{(i,j)} = vol_{mod\text{-}i} - vol_{mod\text{-}j}.$$

In step 150, a brain network is determined for the subject's hippocampus. The brain network is determined by first compiling the similarity measures $Cm_{(i,j)}$ into a squared adjacency matrix, which can also be referred to as a connectivity matrix due to the fact that a similar modified volume of two sub-volumes means that the sub-volumes exhibit a high degree of interconnectivity while a low degree of similarity means that the sub-volumes exhibit a low degree of interconnectivity, as outlined above.

Then, using the interconnectivity matrix, a brain network is built in which each node represents one of the sub-volumes, i.e. one of the anatomical subfields of the subject's hippocampus, and in which an edge or vertex connects each pairing of nodes in the network. The edge is indicative of the determined similarity, i.e. the similarity measure $Cm_{(i,j)}$, between the two nodes connected by the edge and thereby also indicative of the interconnectivity of the two anatomical subfields represented by the nodes. Building the brain network may encompass preparing a graphical representation of the nodes and edges but may also, alternatively or additionally, encompass compiling the necessary information for displaying the brain network into a corresponding data block.

Finally, in step 160, the brain network is prepared for further use by performing one or any combination of, storing the brain network in a memory of the computing device (for example in the form of storing the data block containing necessary information for displaying the brain network on a hard disk of the PC), displaying the brain network on a display unit of the computing device (in particular displaying a graphical representation of the nodes and edges on a screen connected to the PC), and sending the brain network to a remote computing device via a transmission unit of the computing device (for example by sending the data block containing necessary information for displaying the brain network to the remote computing device via a data network such as the internet, a LAN, a mobile network, or the like). The remote computing device may be any computing device different from the computing device on which the brain network is to be put to further use, for example in research.

Obviously, the method may be performed using computing devices other than a PC, for example a tablet computer, smartphone or server. In such case, the above explanations apply accordingly, in particular adapted with regard to the hardware available in a given computing device. Further, the method may be performed on more than one computing device with different steps of the method performed in different computing devices.

Following, an exemplary use case of the method for reconstructing a brain network is described.

First, brain imaging may be performed. In the exemplary use case, 476 multiple sclerosis patients (age 35 $\pm$ 10 years, 337 females, disease duration of 16 $\pm$ 14 months) and a control group of 110 healthy subjects (age 34 $\pm$ 15 years, 54 females) were included. Conventional MRI images were acquired, using a 3T scanner with a 32-channel receive-only head coil, according to a standardized imaging protocol. In the exemplary embodiment, the protocol included a sagittal three-dimensional Ti-weighted (Ti-w) magnetization-prepared rapid gradient echo (MP-RAGE) sequence with the following parameters: repetition time (TR) = 1900 ms, echo time (TE) = 2.52 ms, inversion time (TI) = 900 ms, flip angle (FA) = 9°, matrix size = 256 $\times$ 256 mm, field of view (FOV) = 256 $\times$ 256 mm, slice thickness (ST) = 1 mm, voxel size (VS) = 1 $\times$ 1 $\times$ 1 mm³.

Following, MRI-processing may be performed.

In the example, for estimating subfield volumes, the Ti-w images were input into the software Freesurfer 6.0 to first segment and parcellate the brain. A probabilistic atlas, built by manual labelling on a training dataset normalized to the MNI305 space resulting in a point-to-point correspondence between all training subjects was used as parcellation prior for all brain regions. Such atlas provides the probability of each brain region to belong to a given voxel, the probability of each brain region being determined given the classification of neighboring voxels (neighborhood function), and the probability distribution function of voxel intensities, modelled as a normal distribution, for each brain region at each voxel (cf. Fischl et al.: FreeSurfer, Neuroimage, 62(2): 774-81, 2012). Then, newly introduced Ti-w images of a single given subject to be analyzed may be parcellated by normalizing the new image to the common space and incorporating the subject-specific voxel intensities to find the optimal parcellation that maximizes the probability of observing the input data. Further, the hippocampal subfields are segmented in the example by applying a Bayesian inference approach and a probabilistic high-resolution (~0.1 mm isotropic) ex-vivo atlas based on 15 subjects scanned at 7T (manual delineation of the hippocampal substructures) and in vivo MRI data (manual annotation of the adjacent extra-hippocampal structures). The probabilistic atlas of the example has an isotropic resolution of 0.13 mm, making it possible to delineate the subfields to a high degree of accuracy (cf. Iglesias et al.: Alzheimer's Disease Neuroimaging Initiative. A computational atlas of the hippocampal formation using ex vivo, ultra-high resolution MRI: Application to adaptive segmentation of in vivo MRI, Neuroimage, 115: 117-37, 2015). In the exemplary use case, the FreeSurfer automated hippocampal subfield segmentation has demonstrated high accuracy and reliability within and across healthy and disease populations (cf. Whelan et al.: Heritability and reliability of automatically segmented human hippocampal formation subregions, Neuroimage, 128: 125-37, 2016), and high stability within and across scanner platforms (cf. Brown et al.: Test-retest reliability of FreeSurfer automated hippocampal subfield segmentation within and across scanners, NeuroImage 2020: 116563, 2020).

The preprocessing resulted in 12 hippocampal subfields: hippocampal tail, cornu ammonis 1 (CA1), CA2/3, CA4, subiculum, presubiculum, the granule cell layer of the dentate gyrus (DG), the hippocampus-amygdala transition area (HATA), the parasubiculum, the molecular layer of the hippocampus, the fimbria and the hippocampal fissure. All segmentations were visually checked for quality by an expert in hippocampal segmentation and none was excluded.

Following delineation of each subject's Ti-w MRI hippocampus, with the software of preference (Freesurfer in the exemplary use case), every hippocampal subfield volume was adjusted for variation in total intracranial volume (TIV), age, sex and center by fitting a general linear regression model with all variables and extracting the model residuals as modified or adjusted volume values. By adjusting each region volume for these confounds, independence from individual effects of age heterogeneity, scanner type, and head size was achieved.

Following such adjustment, morphological hippocampal networks for each participant were re-constructed. For this, nodes were assumed to represent hippocampal subfields and edges were assumed to represent the morphometric similarity between each pair of subfields. Then, the inter-nodal network edge similarity was estimated as the absolute difference between the modified or adjusted volumes in every pair of subfield regions according to the formula

$$Cm_{(i,j)} = 1 \,/\, (1 + d_{(i,j)}).$$

Herein, $d_{(i,j)}$ is the morphometric difference between regions i and j which is determined according to the formula

$$d_{(i,j)} = vol_{adj\text{-}i} - vol_{adj\text{-}j}.$$

Next, the resulting edge similarity measure was compiled into a squared adjacency matrix, also called connectivity matrix, representing the connectivity strength across network regions, which can be used to evaluate network topology and evidence regions with connectivity abnormalities related to disease condition.

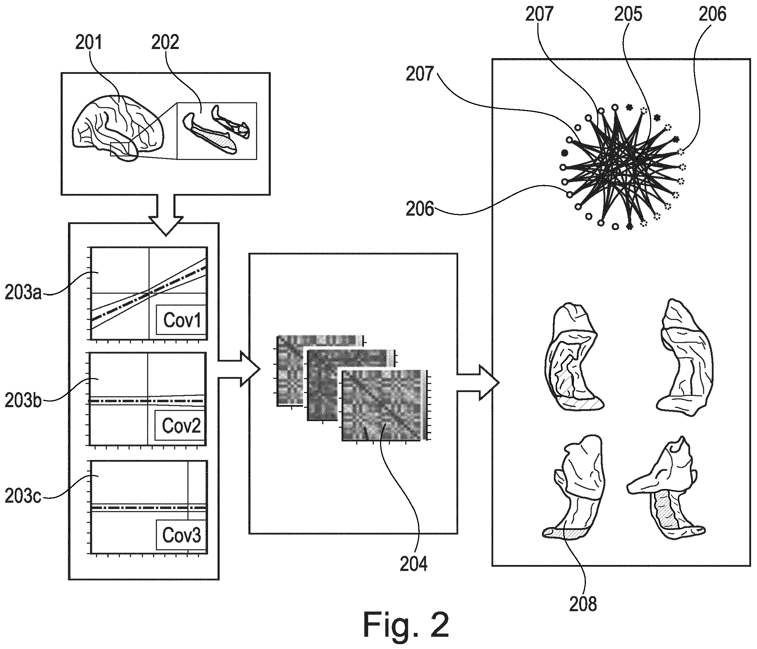

Fig. 2 shows output generated by a method for reconstructing a brain network. Based on Ti-w images of a subject's brain 201, a volume model 202 of the subject's hippocampus is determined. The volume model 202 is segmented into sub-volumes corresponding to anatomical subfields of the hippocampus, distinguished by different colors in the volume model 202. Thereby, regional morphometric values are extracted from the Ti-w images.

Then, adjustment for potential confounding variables is performed, applying a corresponding linear regression 203a, 203b, 203c in line with the different confounding variables. Following, the proposed similarity index is computed and the similarity of each pair of regions is stored into a connectivity matrix 204 and used to evaluate the network topology and search regions of particular disconnection vulnerability.

Further shown in Fig. 2 is a brain network 205 comprising nodes 206 representing the sub-volumes and edges 207 representing the connectivity of the sub-volumes represented by the nodes 206 connected by a given edge 207. Herein, the connectivity is indicated by a color of the edge 207 according to a color gradient (white to blue depicted in grey shades in Fig. 2). The nodes are displayed as rings of two different colors (not discernable in the grey scale image of Fig. 2), one color representing a sub-volume in the right hemisphere and the other color representing a sub-volume in the left hemisphere of the subject's brain. Brain regions determined as clinically significant based on their connectivity are indicated by the corresponding node 206 being represented by a filled ring.

Further, a three-dimensional representation of the hippocampus (208) is output in which the different sub-volumes corresponding to the different anatomical subfields of the hippocampus are displayed in different colors (not shown in Fig. 2) on a color gradient according to their significance. Herein, the significance corresponds to a degree of variation of the determined connectivity for a given sub-volume from a reference connectivity (e.g. a mean or medium connectivity) of the corresponding sub-volume for the population to which the subject belongs.

In a use case according to an exemplary embodiment, the method according to the disclosure was applied to MRI data from 400 patients diagnosed with relapsing-remitting multiple sclerosis (RRMS) and 110 age- and gender-matched healthy controls. The dataset consisted of MRI data at two different time points, namely study inclusion (baseline) and a follow-up after two years.

Semi-automatic MRI pre-processing was performed with FreeSurfer 6.0 followed by segmentation of the hippocampal subfields as described in more detail above and used to reconstruct individual morphometric networks in line with the method according to the disclosure.

Following reconstruction of individual morphometric networks, the graph theoretical network was applied to compute quantitative measures of network topology, which were then used to 1) test group differences at baseline; 2) test changes in network topology after two years; and 3) search for associations between network topology and cognitive performance.

As a measure for cognitive performance the Multiple Sclerosis Inventory of Cognition (MUSIC) test, known as such in the art, was used. More specifically, subtests that may serve as proxies of hippocampal function were employed, namely: (1) Word List Learning, (2) Interference Word List Learning, and (3) Word List Recall.

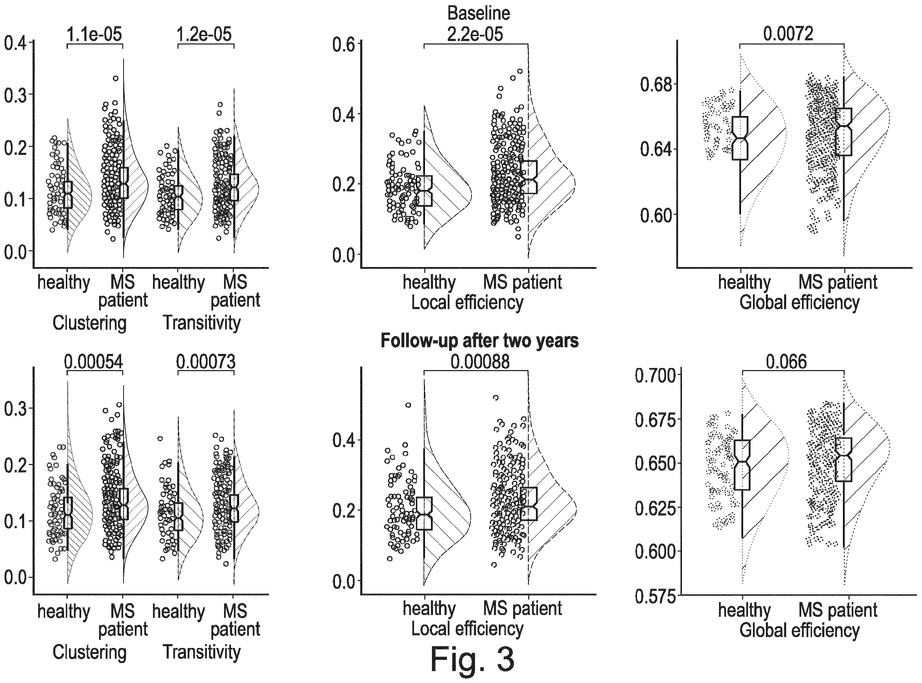

Fig. 3 illustrates the results obtained using a group comparison of network metrics obtained from individual morphometric networks. Four network metrics were computed, including clustering coefficient (CL), transitivity (T), local efficiency (LE) and global Efficiency (GE), which serve to evaluate both segregation and integration of the networks.

At baseline, shown in the upper panel of Fig. 3, the individual hippocampal networks yielded notable differences across groups, where MS patients had increased clustering coefficient and transitivity, indicating more connections with direct network nodes (i.e., subfields). Such results are in concordance with the observed increase in local and global efficiency, the former indicating that the information transfer is becoming less sparse, or in other words, the network is segregating, while the increase in global efficiency largely suggests a restructuration of the whole network and not only in particular nodes. In these analyses, clustering coefficient showed the greatest capability to differentiate among healthy individuals and RRMS patients.

After two years, as illustrated in the lower panel of Fig. 3, the individual networks showed a similar pattern of network reconfiguration except for global efficiency, which no longer showed a significant difference between the groups. Again, the clustering coefficient showed the largest differentiation between groups, and, thus, it was used to evaluate the possible relationship between network topology and cognitive performance.

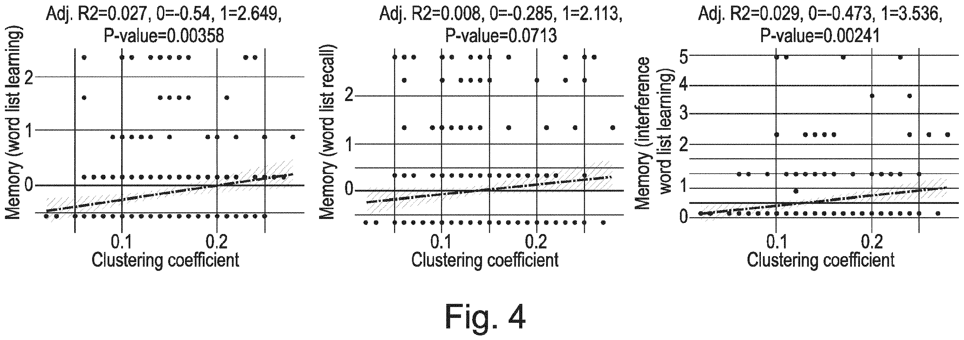

Fig. 4 shows associations between a clustering coefficient and cognitive performance of a subject determined in an exemplary use case. Evaluation of the relationship between network topology re-organization in RRMS and cognitive performance as illustrated in Fig. 4, yielded that the clustering coefficient had a positive association with cognitive performance. Importantly, most of the patients showed a normal cognitive performance despite brain atrophy and clinical disability. Thus, overall, the results suggest that network re-organization in RRMS patients could be firstly compensatory and, possibly, as disease advances the network cannot sustain the functional demands and becomes maladaptive.

In the exemplary embodiments referring to the hippocampus of a subject, given the 12 hippocampal subfields in each hemisphere, the method provides a 24 × 24 fully connected morphological hippocampal network for each individual. However, in other embodiments, the method may be applied to any brain parcellation scheme, in which the resulting connectivity matrix will be of the size N x N, where N represent the number of atlas regions. In this regard, the term fully connected indicates that for each pair of regions, a connectivity value is computed, irrespectively of the particular integrity level of the regions.

The method for reconstructing brain network at the subject level may be performed based on Ti-w images which have a higher spatial resolution than other previously used imaging methods. Such higher spatial resolutions can be achieved without enormously increasing the acquisition time. Voxel resolutions for previously known diffusion and functional MRI data are around 2 to 3 $mm^3$, whereas with Ti-w data, voxel resolutions of 0.7 to 1 $mm^3$ may be feasible. Thereby, additionally, smaller structures may be captured using Ti-w imaging.

The method according to the disclosure may be applied, in particular may be applied without modifications, to compute networks from molecular (i.e., positron emission tomography) or neurotransmitter (i.e., Magnetic Resonance Spectroscopy) signature data. Moreover, it may be applied in subject MRI space as described above, or to images that have been transformed into standard space, for which adjustment for potential head size confounders is not necessary.

According to exemplary embodiments described herein, reconstruction of a brain network may be based on a network of hippocampal subfields. However, the method according to the disclosure may be applied to the whole brain or any other brain sub-system different from or including the hippocampus.

The embodiments of the present invention disclosed herein only constitute specific examples for illustration purposes. The present invention can be implemented in various ways and with many modifications without altering the underlying basic properties. Therefore, the present invention is only defined by the claims as stated below.

LIST OF REFERENCE SIGNS

> 100, 101, 102, 103, 104, 105, 106 — method steps of a method for reconstructing a brain network
> 201 — subject's brain
> 202 — volume model
> 203a, 203b, 203c — linear regression
> 204 — connectivity matrix
> 205 — brain network
> 206 — node
> 207 — edge
> 208 — three-dimensional representation of the hippocampus

**Claims**

1. A method for reconstructing a brain network (205), comprising

> - providing (100), in a memory of a computing device, an image of at least a section of a subject's brain (201);
> - determining (101), in a processing unit of the computing device, a three-dimensional volume model (202) of the section of the subject's brain (201);
> - segmenting (102), by the processing unit, the volume model (202) into a plurality of sub-volumes, each sub-volume corresponding to an anatomical subfield of the subject's brain (201) and having a volume value;
> - for each of the sub-volumes, determining (103), by the processing unit, a modified volume for the sub-volume, wherein determining said modified volume comprises calculating said modified volume based on the volume value of the sub-volume and variables multiplied with constants, wherein the constants are constants determined for a population, comprising a plurality of individuals, to which the subject belongs;
> - for each combination of two sub-volumes, determining (104), by the processing unit, a similarity between the sub-volumes, based on a comparison of the respective modified volume for each of the sub-volumes;
> - determining (105), by the processing unit, a brain network (205) for the section of the subject's brain (201), the brain network (205) comprising nodes (206), each node (206) representing one of the sub-volumes, and edges (207), each edge (207) connecting two nodes (206) and being indicative of the determined similarity between the sub-volumes; and
> - performing (106) at least one of the following steps:

>> - storing the brain network (205) in the memory of the computing device,
>> - displaying the brain network (205) on a display unit of the computing device, and
>> - sending the brain network (205) to a remote computing device via a transmission unit of the computing device.

2. The method according to claim 1, wherein, for each of the sub-volumes, the modified volume is determined according to the following equation:

$$\mathrm{vol}_{mod} = \beta_0 + \beta_1 \cdot x_1 + \dots + \beta_N \cdot x_N + \epsilon$$

wherein

> $\mathrm{vol}_{mod}$ is the modified volume,
> $\beta_0$ is a model section or offset,
> $x_1 \dots x_N$ are the variables,
> $\beta_1 \dots \beta_N$ are coefficients determined by multiplying the volume value of the sub-volume with the respective constants determined for a population for each of the variables, and
> $\epsilon$ is a remaining error,
> preferably wherein some or all of the variables $x_i$ are chosen from the group consisting of the age of the subject, the total volume of the section of the subject's brain, a variable indicative of the sub-volume for which the modified volume is determined, and the gender of the subject.

3. The method according to claim 1 or 2, wherein the constants are obtainable in a statistical analysis and / or using a training algorithm, preferably using linear regression.

4. The method according to any one of the preceding claims, wherein, for each combination of two sub-volumes, the similarity between the sub-volumes is determined according to the following formula:

$$\mathrm{Cm}_{(i,j)} = 1 / (1 + (\mathrm{vol}_{mod\text{-}i} - \mathrm{vol}_{mod\text{-}j}))$$

wherein

Cm$_{(i,j)}$ is the similarity between a sub-volume i and a sub-volume j,
vol$_{mod-i}$ is the modified volume of the sub-volume i, and
vol$_{mod-j}$ is the modified volume of the sub-volume j.

5. The method according to any one of the preceding claims, wherein the section of the subject's brain (201) is the Hippocampus and wherein the sub-volumes are sections of the hippocampus,
preferably wherein each hemisphere of the Hippocampus is segmented into 12 sections such that the volume model is segmented into 24 sub-volumes.

6. The method according to any one of the preceding claims, wherein the image of the section of the subject's brain (201) is an image obtained by MRI of the subject's brain.

7. The method according to any one of the preceding claims, wherein said segmenting is performed using a probabilistic algorithm.

8. The method according to any one of the preceding claims, wherein said storing the brain network (205) comprises compiling the determined similarities between the sub-volumes into a squared adjacency matrix (204).

9. The method according to any one of the preceding claims, wherein said displaying the brain network (205) comprises displaying the edges (207) as connecting lines between the nodes (206), wherein a graphical property of the edges (207) is indicative of the determined similarity between the sub-volumes, the graphical property being selected from an edge thickness, an edge color, an edge length, an edge brightness, and an edge structure.

10. The method according to any one of the preceding claims, wherein

- for each of the sub-volumes, by the processing unit,

- a connectivity measure indicative of the connectivity of the sub-volume to the other sub-volumes is determined,
- the connectivity measure is compared to a representative connectivity measure of the population to which the subject belongs, and
- a significance value for the sub-volume is determined, wherein the significance value is indicative of a deviation of the connectivity measure for the sub-volume from the representative connectivity measure; and

- said displaying the brain network (205) comprises displaying a three-dimensional representation (208) of the section of the subject's brain, wherein each sub-volume is displayed and a graphical property of each sub-volume is indicative of the determined significance value for the sub-volumes, the graphical property being selected from a sub-volume color, a sub-volume brightness, a sub-volume opacity, and a sub-volume structure.

11. A method for monitoring a brain network, comprising

- performing the method of any of the claims 1 to 10 at a first point in time, reconstructing a first brain network;
- performing the method of any of the claims 1 to 10 at a second point in time, the second point in time being later than the first point in time, reconstructing a second brain network; and
- comparing the first brain network to the second brain network,

wherein the first brain network and the second brain network are reconstructed for the same section of the same subject's brain.

12. A computing device comprising a processing unit and a memory, preferably further comprising a display unit and/or a transmission unit, configured to perform the method of any one of the claims 1 to 10.

13. A computer program product comprising instructions which, when the program is executed by a computing device, cause the computing device to carry out the method of any one of the claims 1 to 10.

14. A computer-readable storage medium comprising instructions which, when executed by a computing device, cause the computing device to carry out the method of any one of the claims 1 to 10.

```
┌─────────────────────────────────┐
│   providing image of brain      │
│   (section) in memory of        │──── 100
│   computing device              │
└─────────────────────────────────┘
                │
                ▼
┌─────────────────────────────────┐
│   determining three-dimensional │
│   volume model of hippocampus   │──── 110
└─────────────────────────────────┘
                │
                ▼
┌─────────────────────────────────┐
│   segmenting volume             │
│   module into sub-volumes       │──── 120
└─────────────────────────────────┘
                │
                ▼
┌─────────────────────────────────┐
│   determining modified volume   │
│   for each of the sub-volume    │──── 130
└─────────────────────────────────┘
                │
                ▼
┌─────────────────────────────────┐
│   determining similarity        │
│   between the  sub-volumes      │──── 140
└─────────────────────────────────┘
                │
                ▼
┌─────────────────────────────────┐
│   determining a brain           │
│   network for hippocampus       │──── 150
└─────────────────────────────────┘
                │
                ▼
┌─────────────────────────────────┐
│   preparing brain network       │
│   for further use               │──── 160
└─────────────────────────────────┘
```

# Fig. 1

Fig. 2

Fig. 3

Fig. 4

Europäisches
Patentamt

European
Patent Office

Office européen
des brevets

**EUROPEAN SEARCH REPORT**

Application Number

EP 21 19 9049

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | KONG X-Z ET AL: "Mapping Individual Brain Networks Using Statistical Similarity in Regional Morphology from MRI", PLOS ONE, vol. 10, no. 11, 4 November 2015 (2015-11-04), page e0141840, XP055884753, DOI: 10.1371/journal.pone.0141840 | 1-14 | INV. G16H30/40 G16H50/20 A61B5/055 |
| Y | * p.2 l.28-42; p.3 l.9-10, l.19-28; p.4 l.7, l.13-15, l.22-24 l.32-33; figure 1 * | 1-14 | |
| Y | US 2011/301431 A1 (GREICIUS MICHAEL D [US] ET AL) 8 December 2011 (2011-12-08) * [0006]-[0009], [0017], [0028], [0083], [0103]; Example 1; figures 1-5, 12 * | 1-14 | |
| A | CN 110 459 317 A (UNIV NORTHEASTERN) 15 November 2019 (2019-11-15) * [0006], [0008]-[0010], [0012]-[0015], [0031], [0056] * | 1-14 | |

-/--

TECHNICAL FIELDS
SEARCHED (IPC)

G16H
A61B

The present search report has been drawn up for all claims

| Place of search | Date of completion of the search | Examiner |
|---|---|---|
| Munich | 14 February 2022 | Werner, Andreas |

EPO FORM 1503 03.82 (P04C01)

page 1 of 2

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LU C-F ET AL: "Investigation of differences on cortical connectivity between patients with major depressive disorder and normal subjects using MR diffusion tensor imaging", BIOINFORMATICS AND BIOMEDICAL TECHNOLOGY (ICBBT), 2010 INTERNATIONAL CONFERENCE ON, IEEE, PISCATAWAY, NJ, USA, 16 April 2010 (2010-04-16), pages 79-83, XP031685698, ISBN: 978-1-4244-6775-4 * abstract * * p.81, section "Graph theoretical analysis of brain networks"; p.83 col.1 par.2; figure 3 * | 1-14 | |
| A | CN 112 002 428 A (UNIV TIANJIN MEDICAL) 27 November 2020 (2020-11-27) * [0002], [0009], [0011]; figure 1 * | 1-14 | |
| | | | **TECHNICAL FIELDS SEARCHED (IPC)** |

The present search report has been drawn up for all claims

| Place of search | Date of completion of the search | Examiner |
|---|---|---|
| Munich | 14 February 2022 | Werner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 21 19 9049**

This annex lists the patent family members relating to the patent documents cited in the above-mentioned European search report.
The members are as contained in the European Patent Office EDP file on
The European Patent Office is in no way liable for these particulars which are merely given for the purpose of information.

**14-02-2022**

| Patent document cited in search report | | Publication date | Patent family member(s) | Publication date |
|---|---|---|---|---|
| US 2011301431 | A1 | 08-12-2011 | NONE | |
| CN 110459317 | A | 15-11-2019 | NONE | |
| CN 112002428 | A | 27-11-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GREICIUS et al.** Resting-state functional connectivity reflects structural connectivity in the default model network. *Creb. Cortex,* 2009, vol. 19, 72-78
- **BATALLE et al.** Normalization of similarity-based individual brain networks from gray matter MRI and its association with neurodevelopment in infants with intrauterine growth restriction. *Neuroimage,* 2013, vol. 83, 901-911
- **KONG et al.** Mapping individual brain networks using statistical similarity in regional morphology from MRI. *PLoS ONE,* 2015, vol. 10, e0141840
- **LI et al.** Construction of individual morphological brain networks with multiple morphometric features. *Front. Neuroanat.,* 2017, vol. 11, 34
- **SEIDLITZ et al.** Morphometric Similarity Networks Detect Microscale Cortical Organization and Predict Inter-Individual Cognitive Variation. *Neuron,* 2018, vol. 97 (1), 231-247.e7
- **TIJMS et al.** Similarity-based extraction of individual networks from gray matter MRI scans. *Cereb. Cortex,* 2012, vol. 22, 1530-1541
- **FISCHL et al.** *FreeSurfer, Neuroimage,* 2012, vol. 62 (2), 774-81
- **IGLESIAS et al.** Alzheimer's Disease Neuroimaging Initiative. A computational atlas of the hippocampal formation using ex vivo, ultra-high resolution MRI: Application to adaptive segmentation of in vivo MRI. *Neuroimage,* 2015, vol. 115, 117-37
- **WHELAN et al.** Heritability and reliability of automatically segmented human hippocampal formation subregions. *Neuroimage,* 2016, vol. 128, 125-37
- **BROWN et al.** Test-retest reliability of FreeSurfer automated hippocampal subfield segmentation within and across scanners. *NeuroImage 2020,* 2020, 116563